# EUROPÄISCHE PATENTSCHRIFT

(11) **EP 0 760 357 B1**
(45) Veröffentlichungstag und Bekanntmachung des Hinweises auf die Patenterteilung: **22.12.1999**
(21) Anmeldenummer: 96113230.5
(22) Anmeldetag: 19.08.1996
(51) Int. Cl.: C07C 67/307, C07C 69/78, C07B 39/00

(54) **Verfahren zur Herstellung von 4-Brommethyl-3-methoxybenzoesäure-estern**
Process for preparing 4-bromomethyl-3-methoxy benzoique acid esters
Procédé pour la préparation d'ésters de l'acide benzoique bromométhyle-4, méthoxy-3

(30) Priorität: 24.08.1995 DE 19531164
(43) Veröffentlichungstag der Anmeldung: 05.03.1997
(73) Patentinhaber: Clariant GmbH, 65929 Frankfurt am Main (DE)
(72) Erfinder: Regnat, Dieter, Dr., 65817 Eppstein (DE); Kleiner, Hans-Jerg, Dr., 61476 Kronberg (DE)

(56) Entgegenhaltungen:
- EP-A- 0 595 150
- ANGEW. CHEMIE INT.ED., Bd. 19, Nr. 6, 1980, Seiten 464-465, XP002018765 W. OFFERMANN; F. VÖGTLE: "Brominations with N-Bromosuccinimide: Solvent and Selectivity"
- CHEM. BER., Bd. 114, 1981, Seiten 1048-1064, XP000608479 K. BÖCKMANN, F. VÖGTLE: "Intraanular phenyl-substituierte Phane - Synthese und dynamische Stereochemie"
- J. MED. CHEM., Bd. 33, 1990, Seiten 1771-1781, XP002018766 F.J.BROWN ET AL.: "Evolution of a Series of Peptidoleukotriene Antagonists"
- W. OFFERMANN: "Aromatic Side Chain Bromination...", J. ORG. CHEM., Band 44, Nr. 5, Seiten 710 - 713

## Beschreibung

4-Brommethyl-3-methoxy-benzoesäure-ester, insbesondere der Methylester ist eine wichtige Zwischenstufe beispielsweise bei der Herstellung von Peptidoleukotrien Antagonisten (J. Med. Chem. 33 (1990) 1771, US-PS 4 894 386, EP 337 767, EP 337 766, EP 290 145, JP 6 206 465), von entzündungshemmenden Arzneimitteln (US 5 280 039) und Testosteron-5-α-reductase Inhibitoren (EP 519 353).

Der 4-Brommethyl-3-methoxy-benzoesäure-methylester kann durch Seitenkettenbromierung von 4-Methyl-3-methoxy-benzoesäuremethylester hergestellt werden und zwar entweder mit Brom oder mit N-Brom-succinimid. Man erhält das gewünschte Produkt mit einer Ausbeute von 64 bis 95 % (J. Med. Chem. 33 (1990) 1771; J. Med. Chem. 31 (1988) 692; J. Am. Chem. Soc. 101 (1979) 1857). In all diesen Fällen dient Tetrachlorkohlenstoff als Lösemittel, das jedoch ein ausgeprägtes Zellgift mit der Hauptwirkung auf Leber und Niere ist. Der industrielle Einsatz dieses Lösemittels ist deshalb verboten oder stark eingeschränkt. Es stellte sich daher die Aufgabe, ein Verfahren zur Bromierung von 4-Methyl-4-methoxy-benzoesäureestern zu entwickeln, das nicht mit einem so giftigen Lösemittel arbeitet. Diese Aufgabe wurde gelöst durch eine photochemische Reaktion mit N-Bromsuccinimid in Chlorbenzol oder bestimmten Carbonsäureestern wie im folgenden beschrieben. Es war im Hinblick auf den genannten Stand der Technik überraschend, daß man mit diesem neuen Verfahren unter Verzicht auf das problematische Lösemittel Tetrachlorkohlenstoff gleich hohe Ausbeuten des Zielprodukts erhält wie beim Stand der Technik.

Gegenstand der Erfindung ist ein Verfahren zur Herstellung von 4-Brommethyl-3-methoxy-benzoesäureestern der allgemeinen Formel 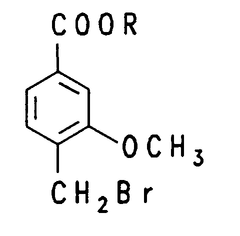 wobei R C₁-C₅-Alkyl bedeutet, dadurch gekennzeichnet, daß man 4-Methyl-3-methoxy-benzoesäureester der allgemeinen Formel 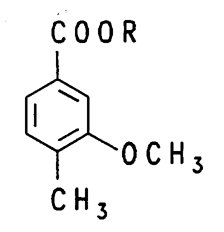 worin R die obengenannte Bedeutung hat, in Chlorbenzol oder einem Ester der Formel

R₂-COOR₃

worin R₂ Wasserstoff oder C₁-C₃-Alkyl und R₃ C₁-C₄-Alkyl bedeuten, mit N-Bromsuccinimid unter Einwirkung von Licht der Wellenlänge 10⁻⁵ bis 10⁻⁸ m bei -10° bis 120°C umsetzt.

Aus dem Reaktionsablauf von Halogenierungen organischer Verbindungen ist bekannt, daß man eine Halogenierung in der Seitenkette eines Aromaten üblicherweise bei recht hohen Temperaturen (Siedehitze) in Gegenwart von Licht (Sonnenlicht) durchführt, während die Halogenierung des Kernes bei tiefen Temperaturen unter Verwendung eines Katalysators erfolgt.

Vor diesem Hintergrund ist es als überraschend anzusehen, daß die erfindungsgemäße Halogenierung unter Einwirkung von Licht bereits bei vergleichsweise niedrigen bis sehr niedrigen Temperaturen nicht in nennenswertem Umfange zu einer Halogenierung des aromatischen Kernes, sondern mit hoher Selektivität zu einer Halogenierung der Methylgruppe führt.

Im allgemeinen spielt die verwendete Menge an Lösungsmittel keine große Rolle. Sie sollte jedoch ausreichend bemessen sein. Im allgemeinen genügt es, 4-Methyl-3-methoxy-benzoesäuremethylester und das Lösungsmittel im Gewichtsverhältnis 1:(3 bis 40), insbesondere 1:(4 bis 20), bevorzugt 1:(5 bis 15) zu verwenden.

Als Lösemittel dient Chlorbenzol oder ein Ester einer aliphatischen Carbonsäure mit 1 bis 4 Kohlenstoffatomen und eines aliphatischen Alkohols mit 1 bis 4 Kohlenstoffatomen oder Mischungen.

Als Lösungsmittel eignet sich beispielsweise Chlorbenzol, Ameisensäuremethylester, Ameisensäureethylester, Essigsäuremethylester, Essigsäureethylester, Essigsäurepropylester, Essigsäurebutylester, Propionsäuremethylester, Propionsäureethylester, Propionsäurepropylester, Propionsäurebutylester, Buttersäuremethylester, Buttersäureethylester, Buttersäurepropylester und Buttersäurebutylester. Mischungen dieser Solventien lassen sich auch verwenden. Gut geeignet als Lösungsmittel sind Chlorbenzol und Ethylacetat.

Üblicherweise setzt man 4-Methyl-3-methoxy-benzoesäureester und N-Bromsuccinimid im Molverhältnis 1:(0,9 bis 1,5) ein. In vielen Fällen genügt es 4-Methyl-2-methoxy-benzoesäuremethylester und N-Bromsuccinimid im Molverhältnis 1:(0,95 bis 1,3), bevorzugt 1:(1,0 bis 1,2) einzusetzen.

Im Verlauf der Halogenierung entsteht aus dem N-Bromsuccinimid Succinimid als Reaktionsprodukt. Das Succinimid kann, gegebenenfalls nach Abkühlen der das Reaktionsgemisch enthaltenden Lösung, durch Filtration abgetrennt werden.

Eine andere Möglichkeit besteht darin, das gebildete Succinimid durch Extraktion mit Wasser abzutrennen. Üblicherweise setzt man hierfür 10 bis 100 Gew.-% Wasser, bezogen auf das Reaktionsgemisch ein.

Eine besonders einfache und zugleich wirksame Methode, Succinimid zu entfernen, besteht darin, das gebildete Succinimid in einem ersten Schritt durch Filtration und in einem zweiten Schritt durch Extraktion mit Wasser aus dem Reaktionsgemisch abzutrennen. Hierbei setzt man vergleichsweise wenig Wasser ein und erhält auch dementsprechend wenig Abwasser.

Das erfindungsgemäße Verfahren wird unter Einwirkung von Licht durchgeführt. Als Lichtquelle kann ein üblicher UV-Strahler, beispielsweise eine Tageslichtlampe, eine dotierte oder undotierte Quecksilberdampflampe oder Niederdruckquecksilberdampflampe, dienen.

Diese Lichtquellen weisen ein Spektrum von 10⁻⁵ bis 10⁻⁸, insbesondere 10⁻⁶ bis 2 x 10⁷ m, auf. Darin sind auch die für die Umsetzung erforderlichen Lichtanteile, insbesondere UV-Anteile, enthalten.

Falls gewünscht, kann man die Umsetzung auch in Anwesenheit eines Radikalbildners durchführen. Es hat sich nämlich gezeigt, daß in einigen Fällen der Zusatz eines Radikalbildner (Radikalstarters) sich zusätzlich zu der Einwirkung von Licht günstig auswirken kann. Geeignete Radikalbildner sind die für eine radikalisch verlaufende Halogenierung üblichen Radikalbildner, beispielsweise organische Peroxide, organische Percarbonsäuren, organische Hydroperoxide oder organische Azoverbindungen.

Beispiele für geeignete Radikalbildner sind Benzoylperoxid, Benzoylperhexadecanoat und Azo-bis-isobutyronitril.

Nichtumgesetzten Radikalbildner kann man, beispielsweise durch Wäsche mit einer wäßrigen Na₂SO₃-Lösung, entfernen.

Man verwendet die Radikalbildner in üblichen Mengen. In der Regel genügt eine Menge von 0,1 bis 5, insbesondere 0,5 bis 2 Gew.-%, bezogen auf 4-Methyl-3-methoxy-benzoesäureester.

Wie eingangs bereits erwähnt, führt man die Bromierung bei -10 bis 120°C durch. In vielen Fällen hat es sich als ausreichend erwiesen, die Bromierung bei -5 bis 100, insbesondere 0 bis 80°C ablaufen zu lassen.

Für eine eventuelle Weiterverarbeitung des nach der Bromierung anfallenden Reaktionsgemisches kann es wünschenswert sein, das ursprünglich in der Stufe der Bromierung verwendete Lösungsmittel durch ein weiteres Lösungsmittel zu ersetzen. Dies ist insbesondere dann notwendig, wenn das ursprüngliche Lösungsmittel sich unter den Bedingungen der Weiterverarbeitung, beispielsweise unter Einwirkung basischer Stoffe, nicht inert verhält. Man setzt in diesem Falle dem Reaktionsgemisch nach Halogenierung ein weiteres Lösungsmittel, das höher als das ursprünglich eingesetzte Lösungsmittel siedet, zu und destilliert anschließend das ursprünglich eingesetzte Lösungsmittel, je nach Wunsch und Bedarf, ganz oder teilweise ab.

Als weiteres Lösungsmittel kommen aromatische Kohlenwasserstoffe, beispielsweise Toluol, o-Xylol, m-Xylol, p-Xylol, Gemische dieser Xylole, Ethylbenzol und/oder Mesitylen und hochsiedende aliphatische Kohlenwasserstoffe, beispielsweise Petrolether mit einem Siedepunkt > 100°C, Decalin, Ligroin und/oder Isooctan in Betracht. Will man den Lösungsmittelaustausch besonders schonend durchführen, kann man das ursprünglich eingesetzte Lösungsmittel als Azeotrop und/oder unter reduziertem Druck abdestillieren.

Beabsichtigt man den 4-Brommethyl-3-methoxybenzoesäureester als Reinprodukt zu isolieren, so entfernt man gegebenenfalls gebildetes Succinimid, wie zuvor beschrieben, durch Filtration und/oder Extraktion mittels Wasser, trocknet das Reaktionsgemisch und filtriert vom Trocknungsmittel ab. Anschließend entfernt man das ursprünglich eingesetzte Lösungsmittel unter reduziertem Druck.
Man erhält ein kristallines Produkt, das gegebenenfalls durch Umkristallisation gereinigt wird. Im allgemeinen ist die Reinheit des erhaltenen Rohprodukts für die weitere Verwendung ausreichend.

### Beispiel 1

18,02 g (0.1 mol) 3-Methoxy-4-methyl-benzoesäure-methylester und 18,68 g (0,105 mol) N-Bromsuccinimid werden in 150 ml Ethylacetat vorgelegt und 4 Stunden bei 0 bis 5°C mit einer UV-Tauchlampe belichtet. Man extrahiert mit 150 ml Wasser, trocknet mit Natriumsulfat, filtriert und engt im Vakuum ein.
Man erhält 25,9 g farblose Kristalle und nach Umkristallisation aus n-Heptan/Ethylacetat im Verhältnis 2:1 24,6 g (95 %) farblose Kristalle mit Schmp. 90°C.

### Beispiel 2

18,02 g (0.1 mol) 3-Methoxy-4-methyl-benzoesäure-methylester und 18,68 g (0,105 mol) N-Bromsuccinimid werden in 150 ml Chlorbenzol vorgelegt und 4 Stunden bei 0 bis 5°C mit einer UV-Tauchlampe belichtet. Man extrahiert mit 150 ml Wasser, trocknet mit Natriumsulfat, filtriert und engt im Vakuum ein.
Man erhält 25,9 g farblose Kristalle und nach Umkristallisation aus n-Heptan/Ethylacetat im Verhältnis 2:1 23,3 g (90 %) farblose Kristalle mit Schmp. 90°C.

## Patentansprüche

1. Verfahren zur Herstellung von 4-Brommethyl-3-methoxy-benzoesäureestern der allgemeinen Formel 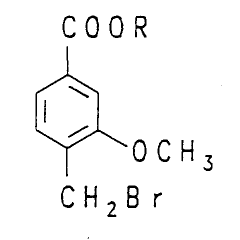 wobei R C₁-C₅-Alkyl bedeutet, dadurch gekennzeichnet, daß man 4-Methyl-3-methoxy-benzoesäureester der allgemeinen Formel 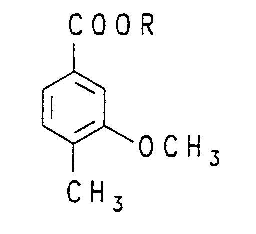 worin R die obengenannte Bedeutung hat, in Chlorbenzol oder einem Ester der Formel
R₂-COOR₃
worin R₂ Wasserstoff oder C₁-C₃-Alkyl und R₃ C₁-C₄-Alkyl bedeuten, oder Mischungen dieser Solventien mit
N-Bromsuccinimid unter Einwirkung von Licht der Wellenlänge 10⁻⁵ bis 10⁻⁸ m bei -10° bis 120°C umsetzt.

2. Verfahren nach Anspruch 1, dadurch gekennzeichnet, daß man die Reaktion in Gegenwart eines Radikalbildners durchführt.

3. Verfahren nach Anspruch 1, dadurch gekennzeichnet, daß man die Reaktion in Gegenwart von Chlorbenzol, Ameisensäuremethylester, Ameisensäureethylester, Essigsäuremethylester, Essigsäureethylester oder Essigsäure-n-propylester durchführt.

## Claims

1. A process for preparing 4-bromomethyl-3-methoxybenzoic esters of the formula 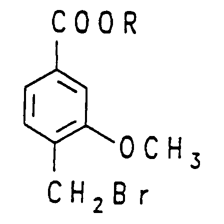 where R is C₁-C₅-alkyl, which comprises reacting 4-methyl-3-methoxybenzoic esters of the formula 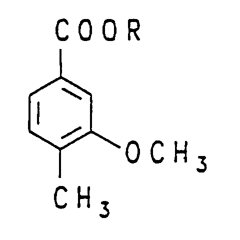 in which R has the abovementioned meaning, in chlorobenzene or an ester of the formula
R₂ - COOR₃
in which R₂ is hydrogen or C₁-C₃-alkyl and R₃ is C₁-C₄-alkyl, or mixtures of these solvents with N-bromosuccinimide with exposure to light of wavelength 10⁻⁵ to 10⁻⁸ m at -10 to 120 C .

2. The process as claimed in claim 1, wherein the reaction is carried out in the presence of a free-radical former.

3. The process as claimed in claim 1, wherein the reaction is carried out in the presence of chlorobenzene, methyl formate, ethyl formate, methyl acetate, ethyl acetate or n-propyl acetate.

## Revendications

1. Procédé pour la préparation d'esters de l'acide 4-bromométhyl-3-méthoxybenzoïque de formule générale : 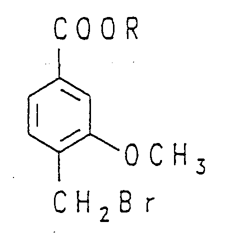 dans laquelle R signifie alkyle en C₁-C₅, caractérisé en ce qu'on fait réagir un ester de l'acide 4-méthyl-3-méthoxy-benzoïque, de formule générale : 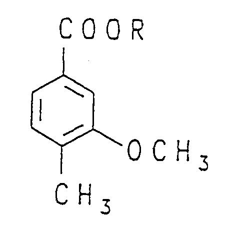 dans laquelle R a la signification indiquée ci-dessus, dans du chlorobenzène ou dans un ester de formule :
R₂-COOR₃
dans laquelle R₂ signifie hydrogène ou alkyle en C₁-C₃, et R₃ alkyle en C₁-C₄, ou des mélanges de ces solvants, avec du N-bromosuccinimide en faisant agir de la lumière de longueur d'ondes 10⁻⁵ à 10⁻⁸ m, à une température de -10° à 120°C.

2. Procédé selon la revendication 1, caractérisé en ce qu'on effectue la réaction en présence d'un formateur de radicaux.

3. Procédé selon la revendication 1, caractérisé en ce que la réaction est effectuée en présence de chlorobenzène, de formiate de méthyle, de formiate d'éthyle, d'acétate de méthyle, d'acétate d'éthyle ou d'acétate de n-propyle.
